Europäisches Patentamt

(19) European Patent Office

Office européen des brevets

(11) Numéro de publication: **0 094 860**
**A1**

## DEMANDE DE BREVET EUROPEEN

(21) Numéro de dépôt: 83400876.5

(22) Date de dépôt: 02.05.83

(51) Int. Cl.³: **B 01 F 3/02**
**A 61 M 16/00**

(30) Priorité: 03.05.82 FR 8207657

(43) Date de publication de la demande:
23.11.83 Bulletin 83/47

(84) Etats contractants désignés:
AT BE CH DE FR GB IT LI LU NL SE

(71) Demandeur: Laboratoire LEJEUNE, SEITZ & AMELINE
66 quai de Jemmapes
F-75010 Paris(FR)

(72) Inventeur: Le Masson, Yves
6, rue Saint-Martin
F-91150 Etampes(FR)

(74) Mandataire: Schrimpf, Robert et al,
Cabinet Regimbeau 26, Avenue Kléber
F-75116 Paris(FR)

(54) Perfectionnements à un dispositif pour mélanger des gaz en écoulement, notamment pour détecter une valeur choisie du rapport des débits des gaz d'un mélange respirable et pour maintenir le rapport à cette valeur.

(57) L'invention concerne un dispositif pour mélanger des gaz en écoulement.

Le dispositif comprend un tube (1) obturé par une bille (2) librement déplaçable dans le tube. Le tube comporte deux entrées de gaz (3 et 4) entre lesquelles peut se déplacer la bille (2). Sur une génératrice du tube, la paroi de celui-ci est munie d'une suite d'orifices (5) et l'ensemble de ces orifices est relié à une sortie commune (6). La paroi du conduit comporte un orifice complémentaire (7) relié par un conduit de gaz (8) à un capteur de débit (9) qui commande une alarme (11).

Application à la détection de l'approche d'une valeur critique du rapport des débits des gaz formant un mélange respirable.

0094860

PERFECTIONNEMENTS A UN DISPOSITIF POUR MELANGER DES GAZ
EN ECOULEMENT, NOTAMMENT POUR DETECTER UNE VALEUR
CHOISIE DU RAPPORT DES DEBITS DES GAZ D'UN MELANGE
RESPIRABLE ET POUR MAINTENIR LE RAPPORT A CETTE VALEUR.   .

L'invention concerne un dispositif pour mélanger des gaz en écoulement.

On a décrit dans le certificat d'utilité n° 78 00719 déposé le 11 janvier 1978 un dispositif pour mélanger des gaz en écoulement, ce dispositif comprenant essentiellement un conduit obturé par un obturateur librement déplaçable dans le conduit sous l'effet d'une pression de gaz, ce conduit comprenant une entrée pour l'un des gaz à mélanger, une autre entrée pour l'autre gaz à mélanger et une pluralité de sorties de gaz échelonnées entre ces deux entrées, l'ensemble de ces sorties étant relié à une sortie commune pour le mélange des gaz.

L'obturateur prend spontanément une position dans le conduit qui dépend du rapport des débits des deux gaz donc du taux du mélange.

Le but de la présente invention est de perfectionner un tel dispositif pour qu'il soit capable de signaler que le taux est à  une valeur choisie et/ou de maintenir le taux à cette valeur. La valeur choisie est par exemple une valeur qui précède et par conséquent qui annonce une valeur critique qu'il ne faut pas atteindre.

On y parvient, selon l'invention, en munissant le conduit d'un orifice complémentaire situé au droit de la position qu'occupe l'obturateur pour cette valeur choisie et en associant à cet orifice un détecteur ou un organe de commande sensible au débit de gaz dans cet orifice.

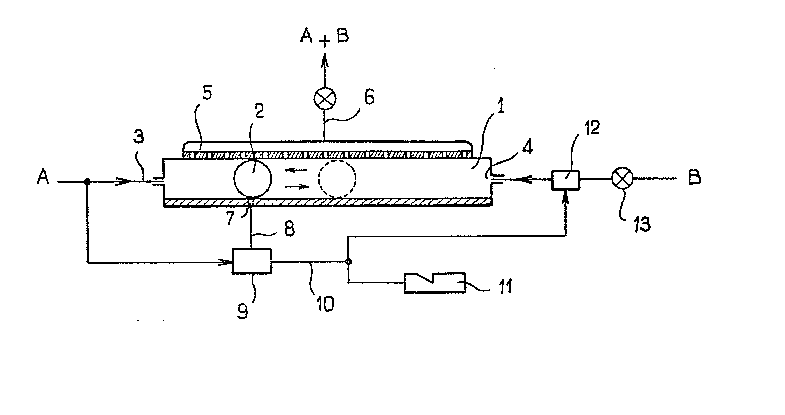

On décrira ci-après un dispositif conforme à l'invention, en référence à la figure unique du dessin joint qui est un schéma de ce dispositif, la description et le schéma étant donnés à titre d'exemple uniquement.

Le dispositif représenté comprend un tube 1 obturé par une bille 2 librement déplaçable dans le tube. Le tube comporte deux entrées de gaz 3 et 4 entre  lesquelles peut se déplacer la bille 1. Sur une génératrice du tube, la paroi de celui-ci est munie d'une suite d'orifices 5 et l'ensemble de ces orifices est relié à une sortie commune 6, comme cela est décrit dans le certificat d'utilité 78 00719.

Les entrées de gaz 3 et 4 sont prévues pour être alimentées respectivement par un gaz A et un gaz B. Le gaz A et/ou le gaz B est un gaz pur ou un mélange de gaz selon les applications du dispositif.

Les débits des deux gaz sont commandés par tout moyen approprié et par exemple, comme décrit dans le certificat d'utilité 78 00719 l'un des gaz A provient d'une alimentation stabilisée en pression et l'autre gaz B alimente l'appareil avec un débit qui est réglé à la demande par un moyen approprié par exemple un robinet à pointeau 13.

Par exemple, lorsque le dispositif est utilisé pour administrer à un malade un mélange respirable de gaz, l'un des gaz est de l'azote et l'autre gaz est de l'oxygène·

Conformément à la présente invention, pour signaler que l'appareil débite un mélange dont le taux correspond à la position de la bille représentée sur la figure, on prévoit sur la paroi du conduit un orifice complémentaire 7 qui sera obturé par la bille lorsque celle-ci se trouve dans la position correspondant à ce taux.

Cet orifice complémentaire de préférence très petit et par exemple de quelques dizièmes de millimètres ne perturbera pas le fonctionnement normal du dispositif.

Cet orifice complémentaire 7 est relié par un conduit de gaz 8 à un capteur de débit 9 qui transforme le signal reçu par le conduit 8, à savoir une interruption de débit de gaz lorsque l'orifice 7 est obturé, en un signal fluidique qui par un conduit 10 commande d'une part une alarme schématisée par un sifflet 11 et, d'autre part, un élément de commande 12 apte à agir sur le débit de l'un des gaz, par exemple le gaz B qui alimente l'entrée 4.

Le capteur 9 est par exemple un capteur pneumatique (cellule logique pneumatique) qui est alimenté par un débit dérivé de l'autre gaz A et piloté par le débit dans le conduit 8, la sortie du capteur fournissant dans la ligne 10 un signal de pression apte à commander d'une part l'alarme 11 et , d'autre part l'organe de commande 12.

L'organe 12 agit sur le débit du gaz B qui n'est pas stabilisé pour provoquer un déplacement de la bille, ce déplacement annulant la commande de l'organe en sorte que la bille revient à la position définie par l'orifice 7, etc...
On obtient donc une oscillation de la bille sur l'orifice 7 et un déclenchement périodique de l'alarme avec une fréquence qui caractérise l'auto-régulation du système.

Il suffit donc de positionner l'orifice 7 à une valeur du taux du mélange inférieure à un seuil indésirable pour être sûr que l'approche de ce seuil sera signalée et que le seuil ne sera pas atteint.

Lorsque l'approche du seuil indésirable est signalée par l'alarme, il suffit à l'opérateur d'agir sur le robinet à pointeau pour modifier le rapport des débits des deux gaz dans le sens qui convient, s'il le désire.

L'invention n'est pas limitée à la réalisation qui a été décrite.

0094860

## REVENDICATIONS

1. Dispositif pour mélanger des gaz en écoulement, ce dispositif comprenant un conduit obturé par un obturateur librement déplaçable dans le conduit sous l'effet d'une pression de gaz, ce conduit comprenant une entrée pour l'un des gaz à mélanger, une autre entrée pour l'autre gaz à mélanger et une pluralité de sorties de gaz échelonnées entre ces deux entrées, l'ensemble de ces sorties étant relié à une sortie commune pour le mélange des gaz, caractérisé en ce que le conduit est muni d'un orifice complémentaire situé au droit de la position qu'occupe l'obturateur pour une valeur choisie du rapport des débits des gaz du mélange et en ce que cet orifice complémentaire est relié à un détecteur ou un organe de commande sensible au débit de gaz dans cet orifice.

2. Dispositif selon la revendication 1, caractérisé en ce que l'orifice complémentaire est relié à un capteur qui détecte l'absence de débit par l'orifice complémentaire et qui fournit un signal de commande d'une alarme.

3. Dispositif selon la revendication 2, caractérisé en ce que le capteur est relié à un organe de commande qui agit sur le débit de l'un des gaz entrant dans le dispositif pour déplacer l'obturateur.

4. Dispositif selon l'une des revendications 2 et 3, caractérisé en ce que le capteur est un capteur pneumatique alimenté par la pression de l'un des gaz et piloté par le débit à travers l'orifice complémentaire.

5. Dispositif selon l'une des revendications 1 à 4, caractérisé en ce que le conduit est un tube et l'obturateur est une bille librement déplaçable dans le tube.

6. Application d'un dispositif selon l'une des revendications 1 à 5 à la production d'un mélange respirable de gaz.

7. Application selon la revendication 6, caractérisée en ce que l'un des gaz alimente le dispositif à une pression stabilisée et en ce que l'autre gaz alimente le dispositif avec ur débit réglable.

0094860

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | DE-A-2 351 718  (STE MINERVE) * Figure * | 1-3 | B 01 F    3/02 <br> A 61 M   16/00 |
| A | DE-A-2 610 416  (K.-H. BAUCH et al.) * Figures 2, 5 * | 1,2 | |
| A | DE-A-2 549 617  (SJUMEK, SJUKVAARDSMEKANIK) * Figure 1 * | 1 | |
| A | US-A-3 817 238  (L.R. MATSON) * Figure 2 * | 1,2 | |
| A | DE-A-1 757 801  (DRÄGERWERK) * Revendication 2; figure 1 * | | |
| A | GB-A-1 344 712  (FORREST MORTON BIRD et al.) * Revendication 2; figure 6 * | | **DOMAINES TECHNIQUES RECHERCHES (Int. Cl. ³)** |
| D,A | FR-A-2 414 362  (Y.R. LE MASSON) * Figure * | 1 | A 61 H   31/00 <br> A 61 M   16/00 <br> A 61 M   17/00 <br> B 01 F    3/02 <br> B 01 F   13/04 <br> B 01 F   15/04 <br> B 63 C   11/32 |

Le présent rapport de recherche a été établi pour toutes les revendications

| Lieu de la recherche | Date d'achèvement de la recherche | Examinateur |
|---|---|---|
| BERLIN | 16-08-1983 | KUEHN P |